# EUROPEAN PATENT APPLICATION

(11) **EP 1 854 478 A1**
(43) Date of publication of application: **14.11.2007**
(21) Application number: 06113895.4
(22) Date of filing: 12.05.2006
(51) Int. Cl.: A61K 39/385, A61K 47/06

(54) **Nicotine-carrier vaccine formulation**

(71) Applicant: Cytos Biotechnology AG, 8952 Schlieren (CH); Ludwig-Maximilians-Universität München, 80539 München (DE)
(72) Inventor: Lang, Rainer, L. Maximilians Universität München, 81371 München (DE); Winter, Gerhard, L. Maximulians Uni. München, 82377 Penzberg (DE); Vogt, Lorenz, 8344 Bäretswil (CH)
(74) Representative: Sperrle, Martin

(57) **Abstract**

The present invention is in the fields of medicine, public health, vaccine and drug formulation. The invention provides composition formulations comprising a nicotine-carrier conjugate and a stabilizer, wherein said stabilizer comprises a non-reducing disaccharide and a non-ionic surfactant. The composition formulations are stable after a long time of storage at room temperature.

## Description

### BACKGROUND OF THE INVENTION

### Field of the Invention

The present invention is in the fields of medicine, vaccine and pharmaceutical formulation. The invention provides formulations comprising a nicotine-virus-like particle conjugate and a stabilizer, wherein said stabilizer comprises a non-reducing disaccharide and a non-ionic surfactant. The lyophilized formulations are stable after a long time of storage at room temperature.

### Related Art

Vaccines for the treatment or prevention of nicotine addiction have recently attracted public attention. These vaccines typically contain nicotine molecules which are covalently bound to a carrier, since nicotine is a low-molecular weight organic compound and not capable of eliciting an immune response by itself. Moreover, since nicotine does not possess suitable functional groups for such a binding to a carrier, the introduction of a linking sequence into the nicotine molecules is typically required. The development of several vaccines has recently been reported, for example in US 5'876'727, US 6'232'082, US 6'656'469 and US 6'932'971. The described conjugates not only vary in the nature of the carrier but also in the nature of the linking sequence and the site where the linking sequence is introduced into the nicotine.

US 6'932'971 describes the coupling of nicotine molecules to a virus-like particle (VLP), that forms an ordered and repetitive nicotine-carrier conjugate and leads to the production of high titer of nicotine-specific antibodies. The same authors have recently shown that a vaccine comprising a virus-like particle of an RNA bacteriophage Qβ to which nicotine molecules are covalently bound by a linking sequence with an ester functionality can be efficacious for smoking cessation in humans (Maurer et al., Eur. J. Immun. 2005 35:2031-40).

The requirement of vaccine compositions to be stable and to minimize or avoid chemical and/or physical degradation implies the need of development of formulations satisfying such requirements.

### SUMMARY OF THE INVENTION

We have now surprisingly found a lyophilized formulation that stabilizes nicotine-virus-like particle conjugates which contain nicotine molecules covalently bound to the virus-like particle by way of a linking sequence, which comprises at least one carboxylic ester functionality. Moreover, we have surprisingly found that this lyophilized formulation is stable over a long period of storage time at room temperature or even at accelerated temperature (40 °C). In addition, the lyophilized formulation of the present invention comprises a simple and economic stabilizer composition due to a minimum number of excipients included therein.

Thus, in one aspect, the invention provides a lyophilized formulation comprising: (i) at least one nicotine-virus-like particle conjugate comprising: (a) a virus-like particle; and (b) at least one nicotine molecule, wherein said at least one nicotine molecule is covalently bound to said virus-like particle by a linking sequence, wherein said linking sequence comprises an ester functionality; and (ii) a stabilizer composition comprising: (c) at least one non-reducing disaccharide, wherein the concentration of said non-reducing disaccharide is from 0.5% to 15% (w/v) in terms of the concentration in the formulation prior to lyophilization; (d) at least one non-ionic surfactant, wherein the concentration of said non-ionic surfactant is from 0.0005% to 0.1% (w/v) in terms of the concentration in the formulation prior to lyophilization; and wherein said stabilizer composition has a pH value from 5.4 to 6.6 prior to lyophilization.

In another aspect, the invention provides a process for making the lyophilized formulation of the invention.

In still another aspect, the invention provides a reconstituted formulation comprising the lyophilized formulation of the invention dissolved and/or suspended in a physiological acceptable solution or in sterile water, preferably in water for injection (WFI). In a further preferred embodiment, the reconstituted formulation further comprises an adjuvant.

### DETAILED DESCRIPTION OF THE INVENTION

Unless defined otherwise, all technical and scientific terms used herein have the same meanings as commonly understood by one of ordinary skill in the art to which this invention belongs.

Adjuvant: The term "adjuvant" as used herein refers to non-specific stimulators of the immune response or substances that allow generation of a depot in the host which when combined with the vaccine and pharmaceutical composition, respectively, of the present invention may provide for an even more enhanced immune response. A variety of adjuvants can be used. Examples include complete and incomplete Freund's adjuvant, aluminum hydroxide and modified muramyldipeptide. Further adjuvants are mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette Guerin) and Corynebacterium parvum. Such adjuvants are also well known in the art. Further adjuvants that can be administered with the compositions of the invention include, but are not limited to, Monophosphoryl lipid immunomodulator, AdjuVax 100a, QS-21, QS-18, CRL1005, Aluminum salts (Alum), MF-59, OM-174, OM-197, OM-294, and Virosomal adjuvant technology. The adjuvants can also comprise a mixture of these substances. VLP has been generally described as an adjuvant. However, the term "adjuvant", as used within the context of this application, refers to an adjuvant not being the VLP used for the inventive formulation, rather in addition to said VLP.

Coat protein: The term "coat protein" and the interchangeably used term "capsid protein" within this application, refers to a viral protein, preferably a subunit of a natural capsid of a virus, preferably of an RNA-bacteriophage, which is capable of being incorporated into a virus capsid or a VLP.

Formulation prior to lyophilization: The term "formulation prior to lyophilization" refers to the liquid formulation of the present invention, which is subject to lyophilization process, typically and preferably within 24 hours, and further typically and preferably within 8 hours, and even more typically and preferably within 2 to 4 hours. The term "lyophilization process" and the term "freeze-drying process" are interchangably used herein and shall be regarded as synonyms herein.

Lyophilized formulation: the term "lyophilized formulation" refers to the composition that is obtained or obtainable by the process of freeze drying of a liquid formulation. Typically and preferably it is a solid composition having a water content of less than 5%, preferably of less than 3%. Preferably, the term "lyophilized formulation" refers to the composition obtained or obtainable by the process for making the lyophilized formulation of the present invention.

Reconstituted formulation: the term "reconstituted formulation" refers to the liquid formulation resulted from the dissolving and/or suspension of the lyophilized formulation in a physiologically acceptable solution.

Linking sequence: the term "linking sequence" as used herein, refers to a molecular entity that covalently links the nicotine molecule to the virus-like particle.

Room temperature: the term "room temperature" as used herein, refers to a temperature from 15°C to 30 °C, preferably from 20°C to 27°C, more preferably 25°C.

Stable: the term "stable" as used herein, refers to the state of the lyophilized formulation of the invention comprising nicotine-VLP conjugates, preferably comprising nicotine-VLP of RNA bacteriophage Qβ conjugates, and even further preferably comprising Nic-Qβ, in which, up to 15 weeks, preferably up to 20 weeks, more preferably up to 25 weeks of storage at room temperature or at accelerated temperature (40°C), (i) the total amount of free nicotine and nicotine derivatives is less than 7%, preferably less than 5%, more preferably less than 3%, even more preferable less than 2% of the total amount of nicotine in the formulation; and (ii) the amount of the sum of nicotine-VLP oligomers and aggregates, preferably the sum of nicotine-VLP of RNA bacteriophage Qβ oligomers and aggregates, preferably the sum of Nic-Qβ oligomers and aggregates, does not increase more than 10%, preferably 7%, more preferably 4% as compared to the amount of the sum of nicotine-VLP oligomers and aggregates, preferably the sum of nicotine-VLP of RNA bacteriophage Qβ oligomers and aggregates, preferably the sum of Nic-Qβ oligomers and aggregates, in the formulation prior to lyophilization. The term "free nicotine and nicotine derivatives", as used herein, refers to nicotine and nicotine derivatives that are not covalently bound to the virus-like particle of the invention. The method to determine the total amount of nicotine as well as the free nicotine or nicotine derivatives, is preferably the RP-HPLC assay as described in EXAMPLE 1 herein. The method to determine the amount of the sum of nicotine-VLP oligomers and aggregates, preferably the sum of nicotine-VLP of RNA bacteriophage Qβ oligomers and aggregates, preferably the sum of Nic-Qβ oligomers and aggregates, is preferable the asymmetrical flow field flow fractionation (AF4) assay as described in EXAMPLE 1 herein, in which fractions containing particles larger than nicotine-VLP monomers and dimers, preferably larger than nicotine-VLP of RNA bacteriophage Qβ oligomers and aggregates, preferably larger than Nic-Qβ monomers and dimers, are combined in calculation.

Oligomer: The term "oligomer", as used in the term "nicotine-VLP oligomer", "nicotine-VLP of RNA bacteriophage Qβ oligomer" and "Nic-Qβ oligomer" refers to the aggregation of at least three and up to ten VLPs or VLPs of Qβ, respectively.

Aggregate: The term "aggregate", as used in the term "nicotine-VLP aggregate", "nicotine-VLP of RNA bacteriophage Qβ aggregate" and "Nic-Qβ aggregate" refers to the aggregation of at least three and up to ten VLPs or VLPs of Qβ, respectively.

Virus particle: The term "virus particle" as used herein refers to the morphological form of a virus. In some virus types it comprises a genome surrounded by a protein capsid; others have additional structures (e.g., envelopes, tails, etc.).

Virus-like particle (VLP), as used herein, refers to a non-replicative or non-infectious, preferably a non-replicative and non-infectious virus particle, or refers to a non-replicative or non-infectious, preferably a non-replicative and non-infectious structure resembling a virus particle, preferably a capsid of a virus. The term "non-replicative", as used herein, refers to being incapable of replicating the genome comprised by the VLP. The term "non-infectious", as used herein, refers to being incapable of entering the host cell. Preferably a virus-like particle in accordance with the invention is non-replicative and/or non-infectious since it lacks all or part of the viral genome or genome function. In one embodiment, a virus-like particle is a virus particle, in which the viral genome has been physically or chemically inactivated. Typically and more preferably a virus-like particle lacks all or part of the replicative and infectious components of the viral genome. A virus-like particle in accordance with the invention may contain nucleic acid distinct from their genome. A typical and preferred embodiment of a virus-like particle in accordance with the present invention is a viral capsid such as the viral capsid of the corresponding virus, bacteriophage, preferably RNA-phage. The terms "viral capsid" or "capsid", refer to a macromolecular assembly composed of viral protein subunits. Typically, there are 60, 120, 180, 240, 300, 360 and more than 360 viral protein subunits. Typically and preferably, the interactions of these subunits lead to the formation of viral capsid or viral-capsid like structure with an inherent repetitive organization, wherein said structure is, typically, spherical or tubular. For example, the capsids of RNA-phages or HBcAgs have a spherical form of icosahedral symmetry. The term "capsid-like structure" as used herein, refers to a macromolecular assembly composed of viral protein subunits resembling the capsid morphology in the above defined sense but deviating from the typical symmetrical assembly while maintaining a sufficient degree of order and repetitiveness. One common feature of virus particle and virus-like particle is its highly ordered and repetitive arrangement of its subunits.

Virus-like particle of an RNA bacteriophage: As used herein, the term "virus-like particle of an RNA bacteriophage" refers to a virus-like particle comprising, or preferably consisting essentially of or consisting of coat proteins, mutants or fragments thereof, of an RNA bacteriophage. In addition, virus-like particle of an RNA bacteriophage resembling the structure of an RNA bacteriophage, being non replicative and/or non-infectious, and lacking at least the gene or genes encoding for the replication machinery of the RNA bacteriophage, and typically also lacking the gene or genes encoding the protein or proteins responsible for viral attachment to or entry into the host. This definition should, however, also encompass virus-like particles of RNA bacteriophages, in which the aforementioned gene or genes are still present but inactive, and, therefore, also leading to non-replicative and/or non-infectious virus-like particles of a RNA phage. Preferred VLPs derived from RNA-bacteriophages exhibit icosahedral symmetry and consist of 180 subunits. Within this present disclosure the term "subunit" and "monomer" are interexchangeably and equivalently used within this context. Preferred methods to render a virus-like particle of an RNA bacteriophage non-replicative and/or non-infectious is by physical, chemical inactivation, such as UV irradiation, formaldehyde treatment, typically and preferably by genetic manipulation.

One, a, or an: when the terms "one", "a", or "an" are used in this disclosure, they mean "at least one" or "one or more" unless otherwise indicated.

In one aspect the invention provides a lyophilized formulation comprising: (i) at least one nicotine-virus-like particle conjugate comprising: (a) a virus-like particle; and (b) at least one nicotine molecule, wherein said at least one nicotine molecule is covalently bound to said virus-like particle by a linking sequence, wherein said linking sequence comprises an ester functionality; and (ii) a stabilizer composition comprising: (c) at least one, preferably one single, non-reducing disaccharide, wherein the concentration of said non-reducing disaccharide is from 0.5% to 15% (w/v) in terms of the concentration in the formulation prior to lyophilization; (d) at least one, preferably one single, non-ionic surfactant, wherein the concentration of said non-ionic surfactant is from 0.0005% to 0.1% (w/v) in terms of the concentration in the formulation prior to lyophilization; and wherein said stabilizer composition has a pH value from 5.4 to 6.6 prior to lyophilization. As it is known in the art that lyophilization of protein composition usually results in a product that is more stable and therefore has a longer shelf-life. Furthermore, the lyophilized formulation has an enhanced stability of the ester functionality present in the nicotine-VLP conjugate and an enhanced stability of the RNA component.

Alternatively in another aspect, the invention provides a liquid formulation comprising: (i) at least one nicotine-virus-like particle conjugate comprising: (a) a virus-like particle; and (b) at least one nicotine molecule, wherein said at least one nicotine molecule is covalently bound to said virus-like particle by a linking sequence, wherein said linking sequence comprises an ester functionality; and (ii) a stabilizer composition comprising: (c) at least one, preferably one single, non-reducing disaccharide, wherein the concentration of said non-reducing disaccharide is from 0.5% to 15% (w/v) in terms of the concentration in said formulation, (d) at least one, preferably one single, non-ionic surfactant, wherein the concentration of said non-ionic surfactant is from 0.0005% to 0.1% (w/v) in terms of the concentration in said formulation; and wherein said stabilizer composition has a pH value from 5.4 to 6.6. Furthermore, the invention provides a formulation obtainable by a method of lyophilization comprising the step of freezing said liquid formulation and drying said liquid formulation.

In another alternative aspect, the invention provides a liquid formulation comprising: (i) at least one nicotine-virus-like particle conjugate comprising: (a) a virus-like particle, and (b) at least one nicotine molecule, wherein said at least one nicotine molecule is covalently bound to said VLP by a linking sequence, wherein said linking sequence comprises an ester functionality; and (ii) a stabilizer composition comprising or consisting of: (c) at least one, preferably one single, non-ionic surfactant, wherein the concentration of said non-ionic surfactant is from 0.0005% to 0.1% (w/v) in terms of the concentration in said formulation; and wherein said stabilizer composition has a pH value from 5.4 to 6.6.

In one preferred embodiment, the liquid or lyophilized formulation of the invention comprises only one carbohydrate, preferably only one sugar. In one preferred embodiment, the liquid or lyophilized formulation of the invention does not comprise an added amino acid. This means no additional amino acid is added to the formulation. However the formulation may comprise trace amount of amino acids due to the degradation of the virus-like particle.

In one preferred embodiment, the liquid or lyophilized formulation of the invention does not comprise a bovine serum albumin or a human serum albumin. In one further preferred embodiment, the formulation of the invention does not comprise any kind of a serum protein. The exclusion of serum advantageously avoids the potential serum contamination problem.

In one preferred embodiment, the liquid or lyophilized formulation of the invention, in particular the lyophilized formulation of the invention, does not comprise sodium chloride. The exclusion of NaCl avoids unnecessary high osmolarity in the formulation. Moreover the exclusion of salt further eliminates the possible adverse effect of salt on protein stability during lyophilization.

In one preferred embodiment, the at least one, preferably one single, non-reducing disaccharide is sucrose or trehalose. In one further preferred embodiment, the non-reducing disaccharide is trehalose.

In one preferred embodiment, the concentration of the at least one, preferably one single, non-reducing disaccharide is from 3% to 15% (w/v), preferably from 5% to 12% (w/v), preferably from 5% to 10% (w/v), preferably from 7.5% to 10% (w/v), preferably 10% (w/v), in terms of concentration in the liquid formulation, or with respect to the lyophilized formulation, in terms of concentration in the formulation prior to lyophilization. The concentration of trehalose expressed in the whole application, unless otherwise explicitly indicated, refers to the concentration of trehalose dihydrate (2H₂O). It is general knowledge for a skilled person to convert between the concentration of trehalose dihydrate and the concentration of water-free trehalose. For example, 10% trehalose dihydrate equals to 9% water-free trehalose.

In one preferred embodiment, the stabilizer composition of the liquid or lyophilized formulation of the invention, preferably of the lyophilized formulation, further comprises at least one, preferably one single, bulking agent. In one further preferred embodiment, the total concentration of said non-reducing disaccharide and said bulking agent is from 0.5% to 15% (w/v), with the proviso that the concentration of said non-reducing disaccharide is at least 0.5% (w/v), in terms of the concentration in the liquid formulation, or with respect to the lyophilized formulation, in terms of concentration in the formulation prior to lyophilization. In one still further preferred embodiment, the total concentration of said at least one, preferably one single, non-reducing disaccharide and said at least one, preferably one single, bulking agent is from 3% to 8% (w/v), preferably from 4.5% to 6% (w/v) in the liquid formulation, or with respect to the lyophilized formulation, in terms of concentration in the formulation prior to lyophilization.

In one preferred embodiment, the bulking agent is mannitol or glycine. In one further preferred embodiment, the bulking agent is mannitol. The inclusion of the bulking agent, preferably mannitol, contributes to the obtaining of a stable cake structure and may allow higher primary drying temperature in the lyophilization process, which advantageously reduces the production cost.

In one preferred embodiment, the pH of the stabilizer composition is from 5.4 to 6.6, preferably from 5.6 to 6.4, preferably from 5.8 to 6.4, preferably from 6.0 to 6.4, preferably 6.2. In one further preferred embodiment, the pH is at 5.4, 5.5, 5.6, 5.7, 5.8, 5.9, 6.0, 6.1, 6.2, 6.3, 6.4, 6.5 and 6.6.

In one preferred embodiment, the non-ionic surfactant is from 0.0025% to 0.02% (w/v), preferably 0.0025%-0.01% (w/v), preferably 0.005% (w/v), in the liquid formulation, or with respect to the lyophilized formulation, in terms of concentration in the formulation prior to lyophilization.

In one preferred embodiment, the non-ionic surfactant is polysorbate 20 or polysorbate 80. In one preferred embodiment, the non-ionic surfactant is polysorbate 20.

Virus-like particles may be of any virus known in the art having an ordered and repetitive structure. Illustrative DNA or RNA viruses, the coat or capsid protein of which can be used for the preparation of VLPs have been disclosed in WO 2004/009124 on page 25, line 10-21, on page 26, line 11-28, and on page 28, line 4 to page 31, line 4. These disclosures are incorporated herein by way of reference.

In one further preferred embodiment, the virus-like particle is of a virus selected from a group consisting of: a) RNA bacteriophages; b) bacteriophages; c) Hepatitis B virus, preferably its capsid protein (Ulrich, et al., Virus Res. 50:141-182 (1998)) or its surface protein (WO 92/11291); d) measles virus (Warnes, et al., Gene 160:173-178 (1995)); e) Sindbis virus; f) rotavirus (US 5,071,651 and US 5,374,426); g) foot-and-mouth-disease virus (Twomey, et al., Vaccine 13:1603 1610, (1995)); h) Norwalk virus (Jiang, X., et al., Science 250:1580 1583 (1990); Matsui, S.M., et al., J. Clin. Invest. 87:1456 1461 (1991)); i) Alphavirus; j) retrovirus, preferably its GAG protein (WO 96/30523); k) retrotransposon Ty, preferably the protein pl; 1) human Papilloma virus (WO 98/15631); m) Polyoma virus; n) Tobacco mosaic virus; o) cowpea mosaic virus; and p) Flock House Virus; q) Cowpea Chlorotic Mottle Virus; and r) an Alfalfa Mosaic Virus. Methods to produce VLP of Cowpea Chlorotic Mottle Virus, Alfalfa Mosaic Virus and cowpea mosaic virus have been described in US 2005/0260758 and in WO05067478.

In one preferred embodiment, the virus-like particle is of an RNA bacteriophage. In one further preferred embodiment, the RNA-bacteriophage is selected from the group consisting of a) bacteriophage Qβ; b) bacteriophage R17; c) bacteriophage fr; d) bacteriophage GA; e) bacteriophage SP; f) bacteriophage MS2; g) bacteriophage M11; h) bacteriophage MX1; i) bacteriophage NL95; k) bacteriophage f2; 1) bacteriophage PP7 and m) bacteriophage AP205. In one preferred embodiment, the virus-like particle is a virus-like particle of an RNA bacteriophage Qβ. Methods for the production of VLP of an RNA bacteriophage, in particular VLP of bacteriophage Qβ and and VLP bacteriophage AP205 have described at page 37-47 of WO 04009124 and in EXAMPLES 1 and 21 thereof.

In one preferred embodiment, the virus-like particle is of RNA bacteriophage Qβ. In one further preferred embodiment, the virus-like particle is of RNA bacteriophage Qβ is recombinantly expressed in E. coli.

In one preferred embodiment, the nicotine-VLP conjugate is from 0.1mg/ml to 2.5 mg/ml in the liquid formulation, or with respect to the lyophilized formulation, in terms of concentration in the formulation prior to lyophilization. In one preferred embodiment, the nicotine-VLP conjugate is from 0.2mg/ml to 2mg/ml in the liquid formulation, or with respect to the lyophilized formulation, in terms of concentration in the formulation prior to lyophilization. In another preferred embodiment of the present invention, the concentration of the nicotine-VLP conjugate in the liquid formulation, typically and preferably in terms of concentration in the formulation prior to lyophilization, is 0.2mg/ml, 0.6mg/ml, 1.0mg/ml or 2mg/ml. In again another preferred embodiment of the present invention, the concentration of the nicotine-VLP conjugate in the liquid formulation, typically and preferably in terms of concentration in the formulation prior to lyophilization, is 0.2mg/ml or 0.6mg/ml, preferably 0.2mg/ml.

Several linking sequences comprising a carboxylic ester functionality with which the nicotine molecules can be covalently bound to a carrier have been described in US 5876727, US 6656469 and US 6932971. These specific teachings are incorporated herein by way of reference. Linking sequences usable for the present invention and comprising an ester functionality, are, for example, the linking sequences termed CJ2, CJ2.1, CJ2.2, CJ2.3, CJ4, CJ4.1, CJ5, CJ5.1, CJ8, CJ8.1, CJ9 and CJ11 as disclosed in column 17 of US5876727.

In one preferred embodiment of the present invention, the linking sequence comprises A-X-CO(O)-Y-Z-B, wherein A represents the nicotine molecule and wherein B represents the virus-like particle, and wherein X=(CH2)m with m=1-4, Y=(CH2)n with m=1-8, and Z= C(O).

In a very preferred embodiment, the linking sequence comprises, consists essentially of, or consists of: CH₂OCO(CH₂)nCO, wherein n=1-8, preferably n=1-4, preferably n=1 or 2, and more preferably n=2. In again a very preferred embodiment, the linking sequence consists of A-CH₂OCO(CH₂)₂CO-B, wherein A represents said nicotine molecule and wherein B represents said virus-like particle.

The linking sequence can either be covalently bound to the pyridine or the pyrrolidine ring of the nicotine molecule. Examples hereto are, in particular, disclosed in US 5876727, US 6656469 and US 6932971. In a very preferred embodiment, said linking sequence is covalently bound to the 3' position of said nicotine molecule.

The totality of the covalently bound nicotine molecules are either present in the same absolute configuration, i.e. all nicotine molecules have the (*R*)-configuration or all nicotine molecules have the naturally occurring (*S*)-configuration, or they are present in any mixture thereof. Preferably, the nicotine molecules are covalently bound such as about an equal mixture or an equal mixture of both the (*R*)-configuration and the naturally occurring (*S*)-configuration is present. In a very preferred embodiment, the nicotine-VLP conjugate comprised by the inventive formulations is obtainable or obtained by using a racemic mixture of nicotine molecules or nicotine derivatives, typically and preferably by using a racemic mixture of nicotine molecules or nicotine derivatives comprising the nicotine molecules with said linking sequence covalently bound thereto, for the coupling reaction to the virus-like particle leading to the nicotine-virus-like particle conjugate in accordance with the invention.

In one preferred embodiment, the nicotine-VLP conjugate, preferably nicotine-VLP of RNA bacteriophge Qβ, preferably NicQβ is formed from the starting material O-succinyl-3'-hydroxymethyl-nicotine and the starting material VLP of Qβ.

In one preferred embodiment, the stabilizer composition comprises a buffering agent selected from sodium phosphate, potassium phosphate and histidine/histidine HCl. In one further preferred embodiment, the concentration of the buffering agent is from 10-20mM in terms of concentration in the liquid formulation, or with respect to the lyophilized formulation, in terms of concentration in the formulation prior to lyophilization. In one further preferred embodiment, the buffering agent is sodium phosphate or potassium phosphate, preferably sodium phosphate.

In one preferred embodiment, the liquid or lyophilized formulation of the invention further comprises sodium chloride from 0 to 90mM, preferably from 0 to 60mM, more preferably from 0 to 30mM. Primarily the inclusion of sodium chloride is to stabilize the liquid solution or to adjust the osmolarity of the liquid or lyophilized formulations of the invention.

In a further very preferred embodiment, the invention provides a lyophilized formulation of the invention that comprises or alternatively consists essentially of or consists of: (i) at least one nicotine-virus-like particle conjugate comprising: (a) a virus-like particle; and (b) at least one nicotine molecule, wherein said at least one nicotine molecule is covalently bound to said virus-like particle by a linking sequence, wherein said linking sequence comprises an ester functionality; and (ii) a stabilizer composition consisting of: (c) at least one, preferably one single, non-reducing disaccharide, wherein the concentration of said non-reducing disaccharide is from 0.5% to 15% (w/v), preferably from 3% to 12% (w/v), in terms of the concentration in the formulation prior to lyophilization; (d) at least one, preferably one single, non-ionic surfactant, wherein the concentration of said non-ionic surfactant is from 0.0005% to 0.1 % (w/v) in terms of the concentration in the formulation prior to lyophilization; (e) a buffering agent, wherein said buffering agent is preferably selected from sodium phosphate, potassium phosphate and Histidine/HistidineHCl; (f) optionally 0-30mM of NaCl in terms of the concentration in the formulation prior to lyophilization; and wherein said stabilizer composition has a pH value from 5.4 to 6.6 prior to lyophilization.

In one alternatively preferred embodiment, the invention provides a lyophilized formulation of the invention comprises or alternatively consists essentially of or consists of: (i) at least one nicotine-virus-like particle conjugate comprising (a) a virus-like particle; and (b) at least one nicotine molecule, wherein said at least one nicotine molecule is covalently bound to said virus-like particle by a linking sequence, wherein said linking sequence comprises an ester functionality; and (ii) a stabilizer composition consisting of: (c) at least one, preferably one single, non-reducing disaccharide, wherein the concentration of said non-reducing disaccharide is from 0.5% to 15% (w/v), in terms of the concentration in the formulation prior to lyophilization; (d) at least one, preferably one single, bulking agent, wherein the total concentration of said non-reducing disaccharide and said bulking agent is from 0.5% to 15% (w/v), with the proviso that the concentration of said non-reducing disaccharide is at least 0.5% (w/v), in terms of the concentration in the formulation prior to lyophilization, (e) at least one, preferably one single, non-ionic surfactant, wherein the concentration of said non-ionic surfactant is from 0.0005% to 0.1% (w/v) in terms of the concentration in the formulation prior to lyophilization; (f) a buffering agent, wherein said buffering agent is preferably selected from sodium phosphate, potassium phosphate and Histidine/HistidineHCl; (g) optionally 0-30mM of NaCl in terms of the concentration in the formulation prior to lyophilization; and wherein said stabilizer composition has a pH value from 5.4 to 6.6 prior to lyophilization.

In one very preferred embodiment, the invention provides a lyophilized formulation of the invention comprises or alternatively consists essentially of or consists of: (i) at least one nicotine-virus-like particle conjugate, preferably at least one nicotine-virus-like particle of an RNA-bacteriphage, preferably at least one nicotine-virus-like particle of RNA-bacteriphage Qβ conjugate, even preferably NicQβ conjugate, comprising (a) a virus-like particle, preferably a virus-like particle of RNA bacteriophage Qβ, wherein the concentration of said conjugate is preferably from 0.1mg/ml to 2mg/ml, preferably from 0.2 mg/ml to 1 mg/ml, in terms of the concentration in the formulation prior to lyophilization; and (b) at least one nicotine molecule, wherein said at least one nicotine molecule is covalently bound to said virus-like particle, preferably to said virus-like particle of RNA bacteriophage Qβ, by a linking sequence, wherein said linking sequence comprises an ester functionality; and (ii) a stabilizer composition consisting of: (c) at least one, preferably one single, non-reducing disaccharide, preferably trehalose, wherein the concentration of said nonreducing disaccharide is from 5% to 12%, preferably 10% (w/v) in terms of the concentration in the formulation prior to lyophilization; (d) at least one, preferably one single, non-ionic surfactant, preferably polysorbate 20, wherein the concentration of said non-ionic surfactant is from 0.005% to 0.1% (w/v), preferably 0.005% (w/v), in terms of the concentration in the formulation prior to lyophilization; (e) a buffering agent, wherein said buffering agent is preferably sodium phosphate or potassium phosphate; and wherein said stabilizer composition has a pH value from 5.6 to 6.2 prior to lyophilization.

A lyophilized formulation comprising: (i) at least one nicotine-virus-like particle conjugate comprising: (a) virus-like particle of RNA bacteriophage Qβ; and (b) at least one nicotine molecule, wherein said at least one nicotine molecule is covalently bound to said virus-like particle by a linking sequence, wherein said linking sequence consists of A-CH₂OCO(CH₂)₂CO-B, and wherein A represents said nicotine molecule and wherein B represents said virus-like particle of RNA bacteriophage Qβ, and wherein said linking sequence is covalently bound to the 3' position of said nicotine molecule; and (ii) a stabilizer composition comprising: (c) one non-reducing disaccharide, wherein said non-reducing disaccharide is trehalose, and wherein the concentration of trehalose is 10% (w/v) in terms of the concentration in the formulation prior to lyophilization; (d)one non-ionic surfactant, wherein said non-ionic surfactant is polysorbate 20, and wherein the concentration of polysorbate 20 is 0.005% (w/v) in terms of the concentration in the formulation prior to lyophilization; and wherein said stabilizer composition has a pH value of 6.2 prior to lyophilization.

A lyophilized formulation comprising: (i) at least one nicotine-virus-like particle conjugate comprising: (a) virus-like particle of RNA bacteriophage Qβ; and (b) at least one nicotine molecule, wherein said at least one nicotine molecule is covalently bound to said virus-like particle by a linking sequence, wherein said linking sequence consists of A-CH₂OCO(CH₂)₂CO-B, and wherein A represents said nicotine molecule and wherein B represents said virus-like particle of RNA bacteriophage Qβ, and wherein said linking sequence is covalently bound to the 3' position of said nicotine molecule; and (ii) a stabilizer composition consisting essentially of or consisting of: (c) one non-reducing disaccharide, wherein said non-reducing disaccharide is trehalose, and wherein the concentration of trehalose is 10% (w/v) in terms of the concentration in the formulation prior to lyophilization; (d) one non-ionic surfactant, wherein said non-ionic surfactant is polysorbate 20, and wherein the concentration of polysorbate 20 is 0.005% (w/v) in terms of the concentration in the formulation prior to lyophilization; (e) one buffering agent, wherein said buffering agent is sodium phosphate, and wherein the concentration of said sodium phosphate is 20mM; and wherein said stabilizer composition has a pH value of 6.2 prior to lyophilization.

In one preferred embodiment, the lyophilized formulation of the invention is stable for at least 15 weeks, preferably at least 25 weeks, at room temperature or even at accelerated temperature (40°C).

In one aspect, the present invention provides a reconstituted formulation comprising the lyophilized composition of the invention dissolved and/or suspended in a physiologically acceptable solution or in sterile water, preferably in water for injection. In another preferred embodiment, the solution is NaCl solution. Preferably the reconstituted formulation has a physiologically acceptable osomolarity value.

In one preferred embodiment, the reconstituted formulation further comprises an adjuvant. In one further preferred embodiment, the adjuvant is aluminium hydroxide hydrated gels or aluminium phosphate hydrated gels.

In one aspect, the present invention provides a process for making the lyophilized formulation of the invention comprising the steps of: (i) freezing the formulation prior to lyophilization by reducing the shelf temperature below -35°C, preferably below -38°C, preferably below -40°C , preferably below -45°C and preferably below -50°C; (ii) primary drying the formulation at the shelf temperature from -45°C to -15°C, preferably from -40°C to - 20°C, preferably from -35°C to -25°C, with the chamber pressure below 0.2 mbar; (iii) secondary drying said formulation at the shelf temperature from 10°C to 30°C with the chamber pressure below 0.2 mbar. The process optionally comprises a step of drying the formulation at the shelf temperature at from -30°C to -15°C, preferably at -20°C, after step (ii), with the chamber pressure below 0.2 mbar.

In one preferred embodiment, the chamber pressure during primary and secondary drying is from 0.020 mbar to 0.2 mbar, preferably from 0.03 to 0.1 mbar, preferably from 0.040 to 0.05 mbar.

In another preferred embodiment, the reducing the shelf temperature is carried out at rate of 0.5°C to 1.0°C/min.

In one preferred embodiment, the process of the invention comprises the steps of: (i) freezing the formulation prior to lyophilization by reducing the shelf temperature below - 40°C, preferably below -50°C; (ii) primary drying the formulation at the shelf temperature - 35°C for at least 10 hours, preferably for 20 hours, with the chamber pressure below 0.2 mbar; (iii) secondary drying said formulation at the shelf temperature from 10°C to 30°C with the chamber pressure below 0.2 mbar. The process optionally comprises a step of drying the formulation at the shelf temperature at -20°C after step (ii), with the chamber pressure below 0.2 mbar.

In one preferred embodiment the process of the invention comprises an additional annealing step, preferably at -10 to -20°C, typically for two to five hours, after the freezing of the formulation by one of the freezing processes as described in the invention. Such annealing step is preferably used when the stabilizer composition of the invention comprises at least one bulking agent, such as mannitol or glycine.

### EXAMPLES

### EXAMPLE 1

### Materials and methods

"NicQβ" - The term "NicQβ", as used herein should refer to at least one nicotine-virus-like particle conjugate comprising (a) a virus-like particle of RNA bacteriophage Qβ; and (b) at least one nicotine molecule, wherein said at least one nicotine molecule is covalently bound to said virus-like particle by a linking sequence, wherein said linking sequence consists of A-CH₂OCO(CH₂)₂CO-B, and wherein A represents said nicotine molecule and wherein B represents said virus-like particle of RNA bacteriophage Qβ, and wherein said linking sequence is covalently bound to the 3' position of said nicotine molecule. NicQβ was produced as described in EXAMPLE 1 of US 6'932'971. NicQβ drug substance was thawed at room temperature.

### Freeze drying protocols

**Table 1: Freeze drying process I.**

| **Step** | **Time [h]** | **Temperature [°C]** | **Pressure [mbar]** |
|---|---|---|---|
| **Loading** | 0:00:00 | 20 | 1013 |
| **Freezing** | 1:10:00 | -50 | 1013 |
| | 3:00:00 | -50 | 1013 |
| **Primary drying** | 0:01:00 | -50 | 0.045 |
| | 0:15:00 | -35 | 0.045 |
| | 20:00:00 | -35 | 0.045 |
| **Secondary drying** | 2:30:00 | -20 | 0.045 |
| | 10:00:00 | -20 | 0.045 |
| | 1:20:00 | 20 | 0.045 |
| | 10:00:00 | 20 | 0.045 |

**Table 2: Freeze drying process II.**

| **Step** | **Time [h]** | **Temperature [°C]** | **Pressure** |
|---|---|---|---|
| **Loading** | 0:00:00 | 20 | 1013 |
| **Freezing** | 1:10:00 | -50 | 1013 |
| | 3:00:00 | -50 | 1013 |
| **Primary drying** | 0:01:00 | -50 | 0.045 |
| | 4:00:00 | -15 | 0.045 |
| | 20:00:00 | -15 | 0.045 |
| **Secondary drying** | 0:01:00 | -15 | 0.007 |
| | 6:00:00 | 40 | 0.007 |
| | 10:00:00 | 40 | 0.007 |

### Reconstitution of the lyophilizates

As known to a skilled person, for some of the analyses described below, the lyophilizates need to be brought into aqueous solution. Thus, the lyophilizates were reconstituted with sterile filtrated water, typically and preferably with sterile filtrated water of a volume to adjust to the total volume of the formulation prior to lyophilization. By way of example, if the formulation prior to the lyophilization process consisted of 0.7 ml per vial, then the preferably formed cake resulting from the lyophilization process is reconstituted in such a volume of sterile water such as the final composition again consists of 0.7 ml.

### Asymmetrical flow field flow fractionation (AF4) measurements to determine VLP aggregates

AF4 measurements were conducted using a Wyatt separation channel with a 350 µm spacer, Eclipse2 separation system (Wyatt Technology Corporation), Agilent 1100 G1310A isocratic pump, Agilent 1100 G1379A degasser, Agilent 1100 G1329A autosampler, Agilent 1100 G1330B thermostat for autosampler, Agilent 1100 G1365B MWD detector, Agilent 1100 G1362A RI detector and Wyatt DAWN EOS MALS detector.

The channel flow was 1.5 ml/min. The cross flow was 2.0 ml/min for 18 minutes, subsequently reduced to 0.15 ml/min in 15 minutes and held for 5 minutes at 0.15 ml/min. In a final step the cross flow was 0.0 ml/min for 5 minutes.

The concentration of VLP was determined at 260 nm with the MWD detector. The Wyatt DAWN EOS MALS detector was used for the determination of the hydrodynamic radius and the molecular weight of VLP species. An amount of around 20 µg VLP from the liquid formulations and reconstituted lyophilizates (reconstituted with water as described above) were injected into the AF4, respectively.

### Differential scanning calorimetry (DSC) measurements to determine glass transistion temprature

DSC measurements were conducted with a Netzsch Differential Scanning Calorimeter 204 Phoenix. Typically, 1 to 25 mg of the sample were weighed into aluminium pans. The pans were then tightly closed with an aluminium lid, using a universal closure press. The reference pan remained empty and was prepared in the same way. The pans were placed in the measuring cell. The cell was flushed with nitrogen. The samples were measured at a heating rate of 10°C/min.

The glass transition temperature, Tg, of the lyophilizates was determined by means of single DSC scans.

### Water content analysis

Moisture content measurements of the lyophilizates were conducted with a coulometric Karl Fischer titrator with a head-space oven (Analytic Jena AG). The lyophilizates were measured right in the 2R glass vial at a head-space temperature of 80°C. The samples were heated in the oven chamber for at least 5 minutes.

### Dynamic light scattering (DLS) measurements to determine homogeneity of formulation

Dynamic light scattering measurements were conducted with a Malvern Zetasizer Nano ZS apparatus. 0.5 to 1.0 ml of the liquid formulation or the reconstituted lyophilizates (reconstituted as described above) were pipetted into UV micro cuvettes Plastibrand and measured applying a validated LMU Munich standard protocol (SOP protein_m_99%). The polydispersity index PI, the proportion of the main NicQb peak and the main peak size applying the volume conversion and intensity models were calculated.

### Light blockage measurements to determine particle contamination and VLP aggregates

Light blockage measurements were conducted with a PAMAS SVSS-C apparatus. The system was flushed with a part of the formulation and subsequently 0.1 - 0.3 ml of the liquid formulation or the reconstituted lyophilizates were assessed for particle contamination. The solution was drawn through the measuring cell and the amount of particles larger than 1, 10 and 25 µm calculated per ml was determined.

### SE-HPLC-RNA integrity

The particles were homogenized in TRI-Reagent (a combination of phenol and guanidine thiocyanate in a mono-phase solution to inhibit of RNase activity) followed by RNA extraction with 1-bromo-3-chloropropane (BCP) - Phase Separation Reagent. Extracted RNA was precipitated with isopropanol and the pellet washed with ethanol. The RNA was then dissolved in DEPC-H₂O and analyzed by HPLC (monitoring effected at A₂₆₀ₙₘ, isocratic elution). The retention time of the extracted RNA was determined relative to a tRNA standard analyzed in the same series.

### RP-HPLC-free nicotine

The soluble nicotine derivatives hydroxymethyl-nicotine (due to hydrolysis of the ester bond) and succinyl-hydroxymethyl-nicotine (due to degradation of the amide bond) were separated from the NicQβ by filtration in spin filters. The flow through was analyzed by RP-HPLC (A₂₆₀ₙₘ - absorption wavelength of nicotine and nicotine derivatives). The concentration of the nicotine derivatives was calculated from the regression of a nicotine standard curve performed in parallel. The values for free nicotine were given in percentage of total nicotine.

### RP-HPLC- total nicotine

The nicotine moiety covalently linked to the Qβ protein was quantitatively cleaved during 3 h incubation at 40°C and pH > 11, after which proteins were precipitated. The hydrolysis product Hydroxymethyl-Nicotine remained in the supernatant and was quantified by RP-HPLC (A_{260 nm}) using a nicotine standard curve, as both the hydrolysis product and nicotine share the same chromophore.

### SE-HPLC- VLP integrity

SE-HPLC is an analytical method to separate different compounds in a sample according to their size. Thus large Qβ particles can be separated from smaller molecules, *e.g.* the Qβ coat protein monomers or nucleic acid fragments and therefore the method was used to confirm the integrity of the VLP. The method was also used to confirm purity of the drug substance. As a control a VLP standard was analyzed with the sample in the same series. Detection was performed at 260 nm. Product-related impurities may be protein aggregates, smaller cleavage products and/or nucleic acids. All these product-related impurities were detected by SE-HPLC, which has been shown to be capable to separate these impurities from the product peak. For detection, a wavelength of 260 nm is used.

### Turbidity measurements

The degree of opalescence of liquid sample solutions was determined using a laboratory turbidimeter (2100 AN, HACH company). Turbidity measurements were performed by ratio turbidimetry which determines the ratio of transmitted light and light scattered by the particles in the sample solution. The instrument was calibrated using formazin turbidity standard suspensions in defined sample cells. For measuring sample volumes of 1-5 ml in smaller test tubes a user-defined calibration curve was established in a range of 0-200 NTU. 1 ml of sample was measured in disposable glass test tubes to which silicon oil has been applied in order to reduce scattering effects caused by the glass.

### VIS-Transmission measurements

The transmission measurements were conducted with a double-beam UV/Visible Spectrophotometer UV1 (Thermo Spectronic). 0.5 to 1.0 ml of the liquid formulation were pipetted into UV micro cuvettes Plastibrand. The transmission at 600 nm was determined.

### EXAMPLE 2

### Effect of pH on the stability of NicQβ

The stability of NicQβ was analyzed at ten different pH's in the range of 4.6 to 8.2. The pH of the bulkware was adjusted by using either a 0.1 N NaOH solution or a 0.1 N H₃PO₄ solution. All samples were diluted to a concentration of 1 mg/ml NicQβ using water. The samples were stored at room temperature up to 14 days. The results are shown in Table 3.

**Table 3: Results - pH stability study NicQß**

| pH | RP-HPLC Content free nicotine derivatives [% of total] at day 7 | SE-HPLC Main peak Rel. peak area at day 7 | PI | DLS Main peak (determined by using the intensity conversion model) [%] |
|---|---|---|---|---|
| 4.6 | 1.7 | 98.5 | 0.26 | 82.9 |
| 5.0 | 1.5 | 98.2 | 0.23 | 89.8 |
| 5.4 | 1.9 | 97.8 | 0.20 | 93.6 |
| 5.8 | 1.6 | 97.3 | 0.21 | 91.8 |
| 6.2 | 3.1 | 97.1 | 0.19 | 94.3 |
| 6.6 | 3.9 | 96.6 | 0.17 | 94.0 |
| 7.0 | 7.2 | 96.3 | 0.14 | 98.9 |
| 7.4 | 11.1 | 91.4 | 0.12 | 100.0 |
| 7.8 | 22.6 | 89.1 | 0.08 | 100.0 |
| 8.2 | 34.8 | 84.0 | 0.10 | 100.0 |

The chemical stability of NicQβ was investigated using RP-HPLC and SE-HPLC methods. Chemical instability of NicQβ results in the degradation of the VLP into monomers or multimers of the Qβ coat protein and/or the disassociation between the nicotine and the VLP of Qβ.

The content of free nicotine derivates increased with increasing pH values. Between pH 4.6 and 6.2 only small increases of free nicotine derivates were detected. Above pH 6.2 the amount of free nicotine derivates increased rapidly over the storage time. Further the VLP integrity was negatively influenced by increasing pH values. At pH equal or higher than 7.4 the integrity of the Qβ capsid decreased drastically after 7 days as measured by SE-HPLC. The relative content of NicQβ at pH 7.0 after 7 days at room temperature was around 96% whereas at pH 7.4 around 91 %.

The physical stability of NicQβ was investigated by light blockage, DLS and VIS-Transmission measurements. Physical stability of Nic-Qβ: the term "physical stability of Nic-Qβ", as used herein, refers to the aggregation of the VLPs of Qβ. All three analytical methods showed that NicQβ tended to aggregate at pH values equal and below 5.8. The DLS measurement showed that the proportion of the main peak decreased while the peak comprising VLP aggregates and oligomers increased and the polydispersity index (PI) increased at pH-values equal and below 5.8. The results obtained by light blockage and VIS-Transmission measurements validated the finding that with a decreasing pH NicQβ tended to aggregation.

### EXAMPLE 3

### Effect of freeze thaw stress conditions on the stability of NicQβ

A total of 36 different formulations of NicQβ were subjected to freeze/thaw cycles. The samples were frozen at - 80°C and thawed at 20°C to 25°C. This freeze/thaw cycles were repeated for 5 times. The formulations were analyzed before and after the freeze/thaw cycles by DLS measurements and by light blockage measurements.

*Effect of trehalose and the addition of polysorbate 20 -* Formulations comprised 0.2mg/ml NicQβ, either 0 or 10% trehalose, pH=6.4, 30mM NaCl with or without 0.005% polysorbate 20. The results of DLS-measurements obtained with the trehalose containing formulations without polysorbate showed a slight decrease of the main peak and an increase of the PI. Thus trehalose led to a slight increase in the aggregation level of NicQβ. However, this effect could be prevented by the addition of polysorbate 20. The light blockage results supported the DLS measurements. A significant lower number of particles > 1 µm was detected in the polysorbate 20 containing formulations after the freeze/thawing as compared to the number of particles > 1 µm in the formulations without polysorbate.

*Effect of different NaCl concentration and the addition of polysorbate 20 -* Formulations comprised 0.2mg/ml NicQβ, 10% trehalose, pH=6.4, various concentrations of NaCl with or without 0.005% polysorbate 20. The results of the DLS measurement showed that NaCl had an influence on the aggregation level of NicQβ after having freeze/thawed. The PI's of the solutions increased with increasing NaCl concentrations after the freeze/thaw cycles. Thus the physical stability of NicQβ was significantly reduced with increasing concentrations of NaCl. However with the addition of polysorbate 20 this physical instability could be compensated for NaCl concentrations equal and below 90 mM. These results were supported by the light blockage measurement. After freeze/thawing, the formulations without polysorbate showed a significant increase of particles larger than 1 µm which indicated a higher amount of aggregates. The addition of polysorbate 20 prevented the aggregation as almost no particles larger than 1 µm were detected.

*Effect of different pH's and the addition of polysorbate 20 -* The effect of pH ranging from 5.4 to 7.2 on the stability of the formulations in the presence or absence of polysorbate 20 were investigated. Formulations comprised 0.2mg/ml NicQβ, 10% trehalose, various pHs, NaCl 30 mM, with or without 0.005% polysorbate 20. The results supported the findings from the pH stability study described in EXAMPLE 2. Already during the preparation of the formulation solutions, the proportion of the main NicQβ peak was decreasing with decreasing pH, as determined by DLS measurements by using the volume conversion model. On the other hand the PI was increasing. The addition of polysorbate 20 prevented the aggregation of NicQβ at tested pH values of 6.4 and 7.2. Furthermore, the addition of polysorbate 20 reduced the aggregation of NicQβ at pH 5.4. In addition to the DLS measurements the results obtained by the light blockage measurement supported these findings. The above described observations made in the course of this pH study by DLS and light blockage measurement were consistent for the NaCl concentrations of 30, 60, 90 and 150 mM.

### EXAMPLE 4

### Influence of varying compositions on the stability of NicQβ during freeze drying

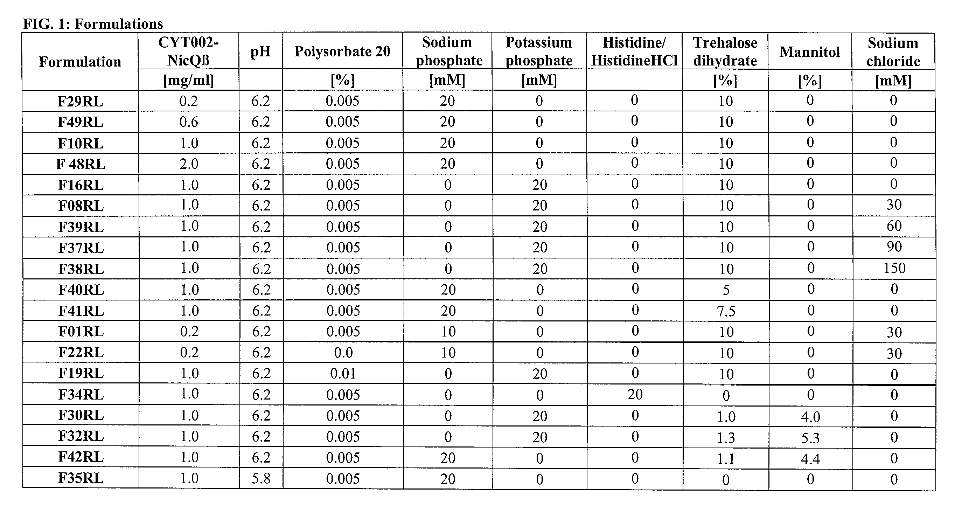

The NicQβ was thawed at room temperature. Various formulations (as shown in FIG. 1) with varying NicQβ, trehalose, polysorbate, NaCl concentrations and with varying buffering system were produced by pipetting the NicQβ into excipient stock solutions. The solutions were stirred on an IKA magnetic stirrer for 5 - 10 minutes. The final drug solutions were sterile filtrated (0.22 µm membrane filter) and were then lyophilized.

Briefly, the drug solutions were filled into sterile 2R glass vials. From the formulations F29RL, F48RL and F49RL 0.7 ml were filled per vial. From the other formulations 0.6 ml were filled per vial. Polydimethylsiloxane and ETFE (Ethylenetetrafluoroethylen)-coated lyophilization stoppers (West Pharmaceutical Services), were placed onto the vials. The vials were transferred into the lyophilization chamber of a Christ Epsilon 2-12 D freeze drier (Martin Christ Gefriertrocknungsanlagen GmbH). Shelf temperature was lowered at 0.5°C to 1.0°C/min to -40°C and held below -40°C for 3 hours. Chamber pressure was then reduced to 0.045 mbar, the shelf was ramped to -35°C at 1°C/min and held for 20 hours. Afterwards the shelf temperature was raised to -20°C at 0.1 °C/min and held for 10 hours. Subsequently the shelf temperature was raised to 20°C at 0.5°C/min and held for 10 hours. The lyophilization chamber was then aerated with filtered dry nitrogen to 800 mbar and the vials were capped in the lyophilization chamber. The vials were removed from the chamber and sealed with Flip-Off® seals. After freeze drying stable lyophilizates were achieved, sufficient cake structure was given.

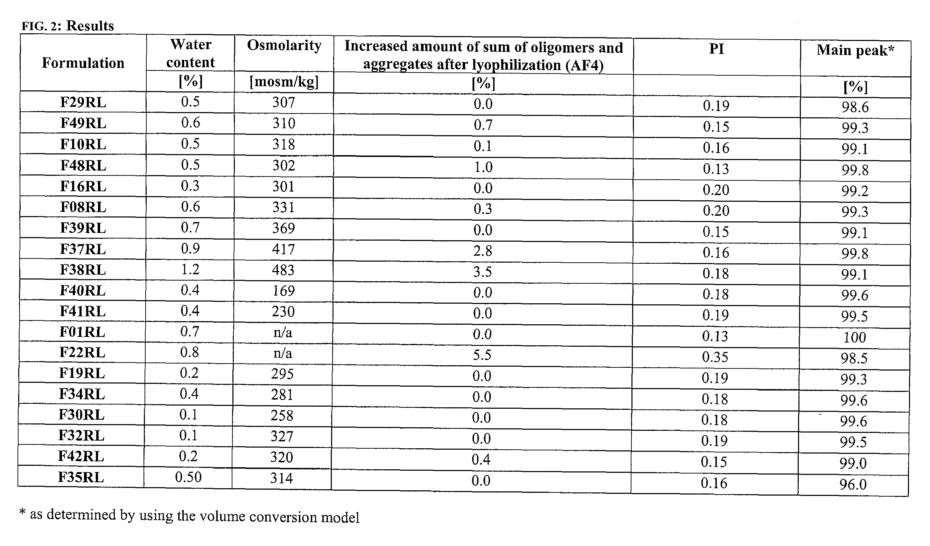

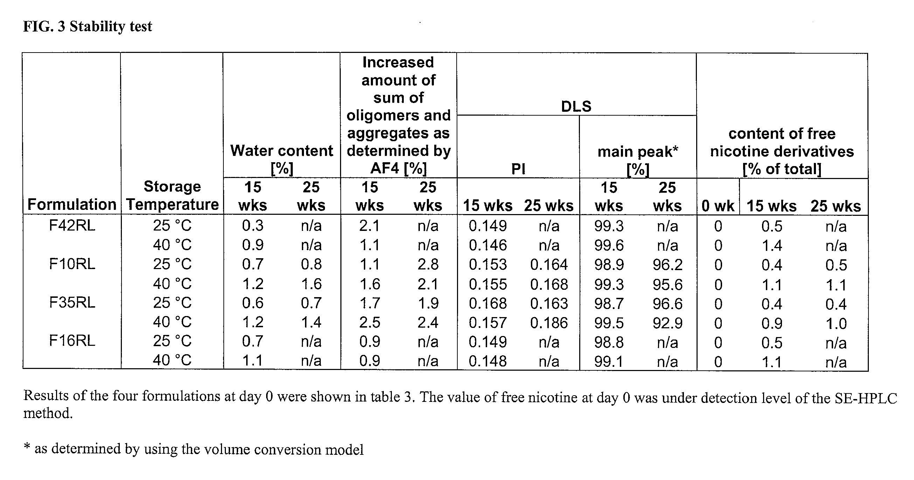

The results are shown in FIG. 2. Thus the lyophilizates from formulations prior to lyophilization with varying Nic-Qb (tested from 0.2mg/ml to 2.0mg/ml), trehalose (tested from 5% to 10%), polysorbate (tested 0.005 to 0.01%), NaCl concentrations (from 0 to 60mM) all had a moisture content less than 1 %. The sum of the amounts of NicQβ-oligomers and NicQβ-aggregates of the above mentioned formulations did not increase more than 1% after freeze-drying in comparison to the formulation before freeze-drying, as analyzed by AF4. The DLS measurements showed that these formulations had polydispersity indices (PI) typically equal and below 0.2 and the proportion of the main NicQβ peak was higher than 98.5% respectively, as determined by using the volume conversion model. The performed analytical measurements led to the conclusion that these above mentioned formulations were capable of stabilizing NicQb in the concentration from 0.20 mg/ml to 2 mg/ml.

F22RL, which did not contain polysorbate 20, had a polydispersity index (PI) value around 0.35. Furthermore, the sum of the amounts of NicQβ-oligomers and NicQβ-aggregates increased about 5.5% after freeze-drying in comparison to the formulation before freeze-drying. These results showed that non-ionic surfactant is necessary for the prevention of VLP aggregation.

F08RL, F39RL, F37RL, F38RL comprised 30mM, 60mM, 90mM and 150mM sodium chloride respectively. While the presence of polysorbate 20 compensated the effect of NaCl (at a concentration equal and below 60mM) on the physical stability of NicQβ, (F08RL and F39RL had no substantial increase of the amounts of NicQβ oligomers and NicQβ-aggregates after lyophilization), the presence of polysorbate 20 only partially compensate the NaCl effect at NaCl concentrations equal and higher than 90 mM as the sum of the amounts of NicQβ-oligomers and NicQβ-aggregates increased 2.8 and 3.5% after freeze-drying. Furthermore the presence of equal or higher than 90mM NaCl resulted in osmolarity values higher than 400 mosm/kg.

### EXAMPLE 5

### Testing of mannitol/trehalose compositions as stabilisers for NicQβ during freeze-drying

**Table 4: Formulations**

| **Formulation** | **NicQ**β | **Polysorbate 20** | **Buffer and molarity** | **Mannitol** | **Trehalose dihydrate** | **pH** |
|---|---|---|---|---|---|---|
| | **[mg/ml]** | **[%]** | **[mM]** | **[%]** | **[%]** | |
| **F30RL** | 1.0 | 0.005 | Potassium phosphate 20 | 4.0 | 1.0 | 6.2 |
| **F32RL** | 1.0 | 0.005 | Potassium phosphate 20 | 5.3 | 1.3 | 6.2 |
| **F42RL** | 1.0 | 0.005 | Sodium phosphate 20 | 4.4 | 1.1 | 6.2 |

Three formulations (F30RL, F32RL, F42RL in FIG. 1) were produced substantially the same as described in EXAMPLE 4. The filling volume of the vials was 0.6ml. The formulations were lyophilized briefly under the following conditions: Shelf temperature was lowered at 1.0°C/min to -50°C and held at -50°C for 3 hours. Chamber pressure was then reduced to 0.045 mbar, the shelf was ramped to -15°C at 0.15°C/min and held for 20 hours. Subsequently the chamber pressure was reduced to 0.007 mbar and the shelf was ramped to 40°C at 0.15°C/min and held for further 10 hours. The lyophilization chamber was then aerated with filtered dry nitrogen to 800 mbar and the vials were capped in the lyophilization chamber. The vials were removed from the chamber and sealed with Flip-Off® seals

The results were partially shown in FIG. 2. After lyophilization stable lyophilizates were achieved and sufficient cake structure was obtained. The moisture content of the three lyophilized formulations was typically below 0.2 %. The osmolarity of the formulations was typically in the range of 250 to 330 [mosm/kg]. The osmolarity increased with increasing trehalose and mannitol concentrations.

The sum of the amounts of NicQβ-oligomers and NicQβ-aggregates did not increase after freeze-drying as analyzed by AF4 measurements. The DLS measurements showed that the three formulations had polydispersity indices (PI) below 0.2 and the proportion of the main NicQβ peak was typically higher than 99 %, as determined by using the volume conversion model.

Light blockage measurements showed that the particle contamination of the reconstituted lyophilizates was typically below 100 particles > 10 µm per ml and the amount of particles > 1 µm was typically below 2000 particles per ml. These results showed that a mixture of trehalose and a bulking agent, such as mannitol, can stabilize the NicQβ during freeze drying.

### EXAMPLE 6

### Stability studies of freeze dried NicQβ formulations

Four formulations F42RL, F35RL, F10RL and F16RL were produced substantially the same as described in EXAMPLE 4. The filling volume of the vials was 0.6ml.

The formulations containing only trehalose were lyophilized under the following conditions: Shelf temperature was lowered at 1.0°C/min to -50°C and held at -50°C for 3 hours. Chamber pressure was then reduced to 0.045 mbar, the shelf was ramped to -35°C at 1°C/min and held for 25 hours. Subsequently the shelf temperature was raised to -20°C at 0.1°C/min and held for 10 hours. Subsequently the shelf temperature was raised to 20°C at 0.5°C/min and held for 10 hours.

The formulations containing both trehalose and mannitol were lyophilized under the following conditions: Shelf temperature was lowered at 1.0°C/min to -50°C and held at - 50°C for 3 hours. Chamber pressure was then reduced to 0.045 mbar, the shelf was ramped to - 15°C at 0.15°C/min and held for 20 hours. Subsequently the chamber pressure was reduced to 0.007 mbar and the shelf was ramped to 40°C at 0.15°C/min and held for further 10 hours.

After lyophilization stable lyophilizates were achieved with all freeze dried formulations and sufficient cake structure was obtained.

The samples were stored at 2 to 8°C, 25°C and 40°C up to 25 weeks. The analytical results were partially shown in FIG. 2. The cake structure of the lyophilizates of all formulations was stable during storage, even at accelerated temperatures. This was observed for all storage conditions and time points. The glass transition temperature of the lyophilizates without mannitol was typically in the range of 60°C to 110°C and was not altered significantly during storage. The osmolarity of the formulations was in the range of 300 to 350 [mosm/kg].

The initial moisture content of the four lyophilized formulations was below 1.0 %. The moisture content of the lyophilizates stored at 2-8°C and 25°C was stable throughout the storage for 25 weeks with an increase of water content of less than 0.5%. The lyophilizates stored at 40°C led to slightly increased moisture contents; the water content after storage was typically below 1.7 % compared to about 1% before storage.

The sum of the amounts of NicQβ-oligomers and NicQβ-aggregates did not increase after freeze drying as analyzed by AF4 measurements. After storage a slight increase of less than 3 % in comparison to formulations prior to lyophilization was observed as analyzed by AF4.

Light blockage measurements showed, that the particle contamination of the reconstituted lyophilizates after storage was typically below 500 particles > 10µm per ml throughout all formulations, storage conditions and time points.

The DLS measurements showed that the reconstituted lyophilizates after storage from the four formulations had polydispersity indices (PI) typically below 0.2 and the proportion of the main NicQβ peak was typically higher than 98.5 % respectively, as determined by using the volume conversion model. This was observed for all formulations, storage conditions and time points.

The IEF, SE-HPLC (RNA integrity), RP-HPLC (total nicotine), Spectrometry (RNA content), turbidity and LDS-Page measurements showed no significant changes of all formulation for all storage conditions and time points. Recombinantly produced virus-like particle of Qβ typically contain host RNA molecules, which are usually in the inner space of the virus-like particles.

The content of free nicotine derivatives was for all formulations, all storage conditions and time points typically below 1.5% of total coupled nicotine, as determined with RP-HPLC measurements.

The SE-HPLC (VLP integrity) measurements showed that the relative content of NicQβ was higher than 97 % throughout all formulations, time points and storage conditions.

The performed analytical measurements led to the conclusion that all formulations are capable for stabilizing NicQβ during lyophilization and following storage, even at accelerated temperatures (40°C).

## Claims

1. A lyophilized formulation comprising:
(i) at least one nicotine-virus-like particle conjugate comprising:
(a) a virus-like particle; and
(b) at least one nicotine molecule,
wherein said at least one nicotine molecule is covalently bound to said virus-like particle by a linking sequence,
wherein said linking sequence comprises an ester functionality; and
(ii) a stabilizer composition comprising:
(c) at least one non-reducing disaccharide, wherein the concentration of said non-reducing disaccharide is from 0.5% to 15% (w/v) in terms of the concentration in the formulation prior to lyophilization;
(d) at least one non-ionic surfactant, wherein the concentration of said non-ionic surfactant is from 0.0005% to 0.1% (w/v) in terms of the concentration in the formulation prior to lyophilization;
and wherein said stabilizer composition has a pH value from 5.4 to 6.6 prior to lyophilization.

2. The lyophilized formulation of claim 1, wherein said non-reducing disaccharide is sucrose or trehalose.

3. The lyophilized formulation of claim 1 or 2, wherein said non-reducing disaccharide is trehalose.

4. The lyophilized formulation of any one of the preceding claims, wherein the concentration of said at least one non-reducing disaccharide is from 3% to 12% (w/v) in terms of the concentration in the formulation prior to lyophilization, and wherein preferably the concentration of said at least one non-reducing disaccharide is 10% (w/v) in terms of the concentration in the formulation prior to lyophilization.

5. The lyophilized formulation of any one of the preceding claims, wherein said stabilizer composition further comprises a bulking agent.

6. The lyophilized formulation of claim 5, wherein the total concentration of said non-reducing disaccharide and said bulking agent is from 0.5% to 15% (w/v) in terms of the concentration in the formulation prior to lyophilization, with the proviso that the concentration of said non-reducing disaccharide is at least 0.5% (w/v) in terms of the concentration in the formulation prior to lyophilization.

7. The lyophilized formulation of claim 5 or 6, wherein said bulking agent is mannitol.

8. The lyophilized formulation of any one of the preceding claims, wherein the concentration of said non-ionic surfactant is from 0.0025% to 0.01 % (w/v), preferably 0.005% (w/v), in terms of concentration in the formulation prior to lyophilization.

9. The lyophilized formulation of any one of the preceding claims, wherein said non-ionic surfactant is polysorbate 20.

10. The lyophilized formulation of any one of the preceding claims, wherein said virus-like particle is a virus-like particle of an RNA bacteriophage, and wherein preferably said virus-like particle is a virus-like particle of RNA bacteriophage Qβ.

11. The lyophilized formulation of any one of the preceding claims, wherein said linking sequence consists of A-CH₂OCO(CH₂)₂CO-B, wherein A represents said nicotine molecule and wherein B represents said virus-like particle.

12. The lyophilized formulation of claim 11, wherein said linking sequence is covalently bound to the 3' position of said nicotine molecule.

13. The lyophilized formulation of any one of the preceding claims, wherein said stabilizer composition further comprising a buffering agent selected from sodium phosphate, potassium phosphate and Histidine/HistidineHCl, and wherein preferably said stabilizer composition further comprising a buffering agent being sodium phosphate.

14. A lyophilized formulation comprising:
(i) at least one nicotine-virus-like particle conjugate comprising:
(a) a virus-like particle of RNA bacteriophage Qβ; and
(b) at least one nicotine molecule,
wherein said at least one nicotine molecule is covalently bound to said virus-like particle by a linking sequence,
wherein said linking sequence consists of A-CH₂OCO(CH₂)₂CO-B, and wherein A represents said nicotine molecule and wherein B represents said virus-like particle of RNA bacteriophage Qβ, and wherein said linking sequence is covalently bound to the 3' position of said nicotine molecule; and
(ii) a stabilizer composition comprising:
(c) one non-reducing disaccharide, wherein said non-reducing disaccharide is trehalose, and wherein the concentration of trehalose is 10% (w/v) in terms of the concentration in the formulation prior to lyophilization;
(d) one non-ionic surfactant, wherein said non-ionic surfactant is polysorbate 20, and wherein the concentration of polysorbate 20 is 0.005% (w/v) in terms of the concentration in the formulation prior to lyophilization;
and wherein said stabilizer composition has a pH value of 6.2 prior to lyophilization.

15. A lyophilized formulation comprising:
(i) at least one nicotine-virus-like particle conjugate comprising:
(a) a virus-like particle of RNA bacteriophage Qβ; and
(b) at least one nicotine molecule,
wherein said at least one nicotine molecule is covalently bound to said virus-like particle by a linking sequence,
wherein said linking sequence consists of A-CH₂OCO(CH₂)₂CO-B, and wherein A represents said nicotine molecule and wherein B represents said virus-like particle of RNA bacteriophage Qβ, and wherein said linking sequence is covalently bound to the 3' position of said nicotine molecule; and
(ii) a stabilizer composition consisting of:
(c) one non-reducing disaccharide, wherein said non-reducing disaccharide is trehalose, and wherein the concentration of trehalose is 10% (w/v) in terms of the concentration in the formulation prior to lyophilization;
(d) one non-ionic surfactant, wherein said non-ionic surfactant is polysorbate 20, and wherein the concentration of polysorbate 20 is 0.005% (w/v) in terms of the concentration in the formulation prior to lyophilization;
(e) one buffering agent, wherein said buffering agent is sodium phosphate, and wherein the concentration of said sodium phosphate is 20mM; and wherein said stabilizer composition has a pH value of 6.2 prior to lyophilization.

16. The lyophilized formulation of any one of the preceding claims, wherein said lyophilized formulation is stable at room temperature for at least 15 weeks, preferably for at least 25 weeks.
